# FASCICULE DE BREVET EUROPEEN

(11) **EP 2 558 089 B1**
(45) Date de publication et mention de la délivrance du brevet: **23.05.2018**
(21) Numéro de dépôt: 11717033.2
(22) Date de dépôt: 05.04.2011
(51) Int. Cl.: A61K 31/366, A61K 31/00, A61P 25/30, A61P 25/32, A61P 25/34, A61P 25/36

(54) **STATINES POUR LA PREVENTION OU LE TRAITEMENT DES ADDICTIONS AUX DROGUES**
STATINE ZUR PRÄVENTION ODER BEHANDLUNG VON DROGENABHÄNGIGKEIT
STATINS FOR THE PREVENTION OR TREATMENT OF DRUG ADDICTIONS

(30) Priorité: 14.04.2010 FR 1001581
(43) Date de publication de la demande: 20.02.2013
(73) Titulaire: Centre National de la Recherche Scientifique, 75016 Paris (FR); Université De Poitiers, 86034 Poitiers Cedex (FR)
(72) Inventeur: SOLINAS, Marcello, F-86000 Poitiers (FR); CHAUVET, Claudia, F-86000 Poitiers (FR); JABER, Mohamed, F-86180 Buxerolles (FR)
(74) Mandataire: Lavoix
(86) Numéro de dépôt international: PCT/IB2011/051457
(87) Numéro de publication internationale: WO 2011/128810

(56) Documents cités:
- EP-A1- 1 745 781
- WO-A1-2004/019947
- WO-A1-2005/061509
- WO-A2-2007/123949
- WO-A2-2007/136607
- FR-A1- 2 882 263
- DATABASE WPI Week 200337 Thomson Scientific, London, GB; AN 2003-382513 XP002591621, & CN 1 186 659 A (UNIV CHONGQING MEDICAL) 8 juillet 1998 (1998-07-08)
- ' Brien ET AL: "Reviews and Overviews Anticraving Medications for Relapse Prevention: A Possible New Class of Psychoactive Medications", Am J PsychiatryAm J Psychiatry, 1 August 2005 (2005-08-01), pages 1423-1431, XP055421701, Retrieved from the Internet: URL:http://ajp.psychiatryonline.org/doi/pd f/10.1176/appi.ajp.162.8.1423 [retrieved on 2017-11-03]

## Description

### Domaine de l'invention

La présente invention concerne des médicaments pour leur utilisation dans la prévention ou le traitement des addictions aux drogues ou dans le sevrage de la consommation de drogues.

### Arrière plan technique

L'augmentation de la consommation de drogues d'addiction, telles que la cocaïne, dans la population générale, et notamment chez les jeunes adultes, rend nécessaire le développement de stratégies facilitant le sevrage et limitant la rechute en cas de sevrage, en particulier en réduisant le désir pour la drogue, plus connu sous la terminologie anglo-saxonne de *craving.*

A l'heure actuelle, deux grands types de traitements des addictions sont proposés, pharmacologiques et non pharmacologiques.

Les traitements non pharmacologiques font essentiellement intervenir la psychothérapie, afin d'identifier les éventuels déséquilibres à l'origine de la prise de drogue et de les corriger, ainsi que de modifier le comportement des individus vis-à-vis de la drogue d'addiction. Toutefois, la dépendance est parfois telle qu'il est nécessaire d'avoir recours à des moyens complémentaires.

Les présents inventeurs ont ainsi proposé, sur la base d'expériences réalisées chez la souris, que des conditions environnementales enrichies, notamment par des stimulations sociales, physiques et intellectuelles, pourraient faciliter le sevrage chez les individus dépendants à la cocaïne (Solinas et al. (2008) Proc. Natl. Acad. Sci. USA 105:17145-17150). Cependant, ce traitement a également ses limites, puisque des travaux récents des inventeurs, confirmés par les travaux d'une autre équipe, indiquent qu'une prise de cocaïne chez un sujet sevré traité par un environnement enrichi pourrait réinstaurer une dépendance à la cocaïne (Chauvet et al. (2009) Neuropsychopharmacology 34:2767-2778 ; Thiel et al. (2009) Int. J. Neuropsychopharmacol. 12:1151-1156).

Il a également été récemment proposé, sur la base d'expériences réalisées chez le rat, qu'une stimulation électrique à haute fréquence du noyau subthalamique, à l'aide d'électrodes intracérébrales, pourrait permettre de diminuer le désir pour la cocaïne (Rouaud et al. (2010) Proc. Natl. Acad. Sci. USA 107:1196-200). Toutefois, si l'invasivité de cette technique est acceptable dans le cas de maladies neuro-dégénératives graves, comme la maladie de Parkinson, cela semble moins justifié dans des cas de dépendance simples à une drogue, et il semble que cette technique devrait être réservée à des cas de dépendance ne pouvant être pris en charge autrement.

S'agissant des traitements pharmacologiques, la pharmacopée disponible est relativement limitée, notamment en ce qui concerne le traitement de la dépendance à la cocaïne. De fait, pour cette dernière, il n'existe à l'heure actuelle aucun véritable traitement pharmacologique de la dépendance. En effet, les traitements pharmacologiques utilisés visent essentiellement à traiter certains effets secondaires du sevrage à la cocaïne, comme la dépression. Cinq composés, approuvés pour d'autres indications, ont bien montré un effet bénéfique au cours d'essais cliniques, à savoir le disulfiram, le modafinil, le propranolol, le topiramate, la vigabatrine et le baclofène, mais ils ne sont pas encore officiellement approuvés pour le traitement du sevrage à la cocaïne (O'Brien (2005) Am. J. Psychiatry 162:1423-1431).

Il reste donc urgent de trouver un traitement alternatif, ou complémentaire, aux traitements actuels pour améliorer la prise en charge de la dépendance aux drogues.

Les statines sont des inhibiteurs de la HMG-CoA réductase, une enzyme de la voie de synthèse du cholestérol. Elles sont principalement indiquées pour réduire la cholestérolémie chez les individus présentant un risque d'événement cardiovasculaire.

### Résumé de l'invention

La présente invention découle de la mise en évidence, inattendue, par les inventeurs, qu'au moins une statine, la simvastatine, administrée de manière chronique (1 mg/kg i.p.) durant une période de sevrage de 20 jours à des rats préalablement rendus dépendants à la cocaïne ou à la nicotine, permettait de réduire de manière significative le désir (ou *craving*) pour la drogue après la période de sevrage, par rapport à des rats témoins ayant reçu un simple véhicule au lieu de la simvastatine.

Ainsi, la présente invention concerne au moins une statine pour son utilisation dans la prévention ou le traitement de l'addiction à une drogue ou dans le sevrage de la consommation d'une drogue chez un individu.

La présente invention concerne également l'utilisation d'au moins une statine pour la préparation d'un médicament destiné à la prévention ou au traitement de l'addiction à une drogue ou dans le sevrage de la consommation d'une drogue chez un individu.

La présente invention concerne également une méthode de prévention ou de traitement de l'addiction à une drogue ou de sevrage de la consommation d'une drogue chez un individu, dans laquelle on administre à l'individu une quantité prophylactiquement ou thérapeutiquement efficace d'au moins une statine.

Dans un mode de réalisation préféré de la statine pour son utilisation définie ci-dessus, de l'utilisation de la statine telle que définie ci-dessus et de la méthode définie ci-dessus, la statine est en combinaison avec au moins un composé additionnel destiné à la prévention ou au traitement de l'addiction à une drogue ou au sevrage de la consommation d'une drogue.

La présente invention concerne également une composition pharmaceutique comprenant à titre de substances actives :
- au moins une statine, et
- au moins un composé additionnel sélectionné dans le groupe spécifié dans la revendication 9, et
- éventuellement un véhicule pharmaceutiquement acceptable, de préférence pour son utilisation dans la prévention ou le traitement ou dans le sevrage de la consommation d'une drogue chez un individu.

La présente invention concerne également des produits comprenant :
- au moins une statine, et
- au moins un composé additionnel sélectionné dans le groupe spécifié dans la revendication 11, en tant que produit de combinaison pour une utilisation simultanée, séparée ou étalée dans le temps, dans la prévention ou le traitement d'une addiction à une drogue ou dans le sevrage de la consommation d'une drogue.

### Description des figures

### Figures 1A et 1B

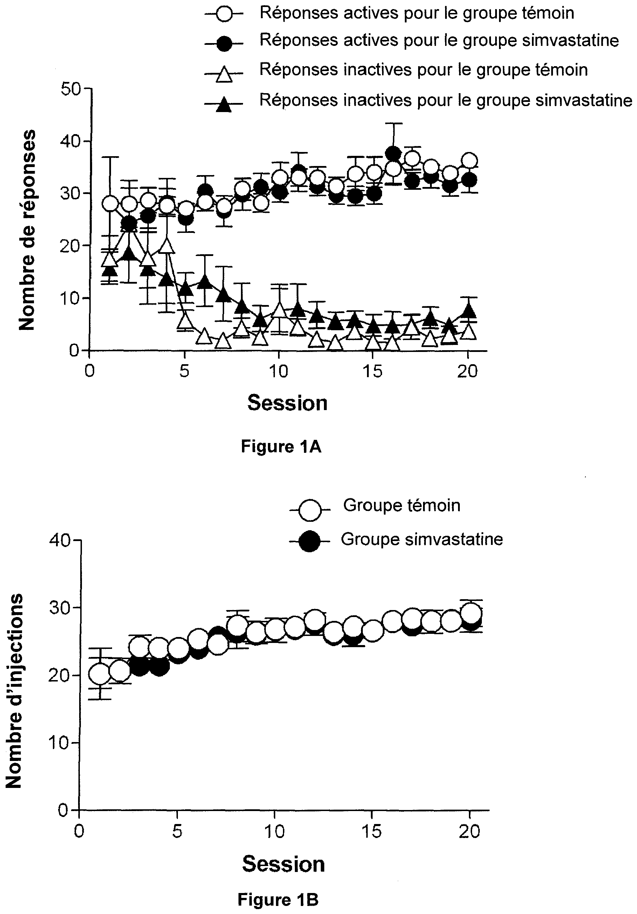

Les figures 1A et 1B représentent les niveaux moyens d'auto-administration de cocaïne chez les rats qui recevront par la suite un traitement chronique d'une solution témoin (Groupe témoin) ou un traitement chronique de simvastatine (Groupe simvastatine). La figure 1A représente le nombre de réponses pour les orifices *(nose-pokes)* actifs et inactifs et la figure 1B représente le nombre d'injections de cocaïne durant les 20 sessions d'auto-administration. Il est à noter que les traitements chroniques de solution témoin ou de simvastatine débutent le jour après la dernière session d'auto-administration et se terminent le jour précédent les tests de comportement de recherche de drogues.

### Figures 2A et 2B

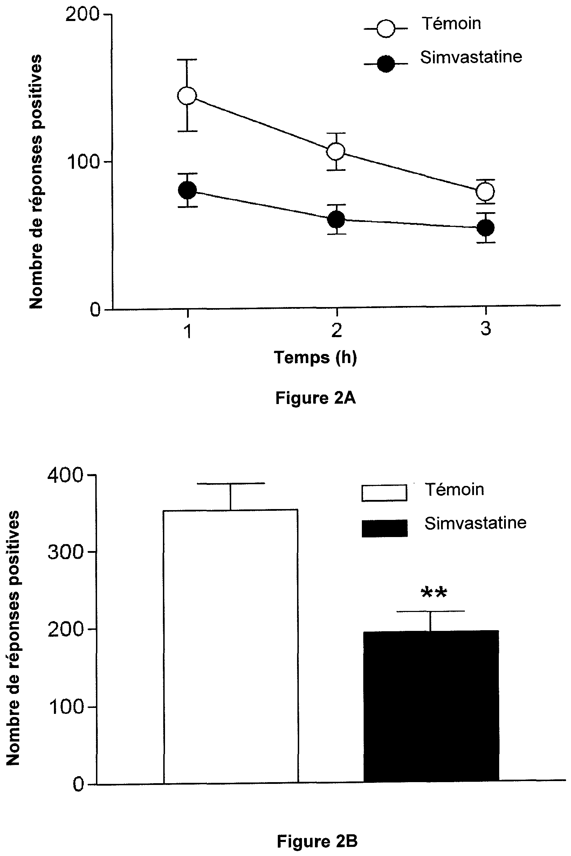

Les figures 2A et 2B représentent la diminution du comportement de recherche de cocaïne chez les rats traités de manière chronique à la simvastatine. Le comportement de recherche de drogues est mesuré par le nombre de réponses dans les orifices (*nose-pokes*) actifs réalisées par les rats durant la session du test d'extinction de 3 h. Les réponses actives ne sont pas renforcées par une injection de cocaïne. La figure 2A représente le nombre de réponses dans les orifices (*nose-pokes*) actifs durant chaque heure du test d'extinction. La figure 2B représente le nombre total de réponses dans les orifices *(nose-pokes)* actifs durant les 3 heures d'extinction. Le symbole ** indique une diminution significative des réponses du groupe simvastatine par rapport au groupe témoin.

### Figures 3A et 3B

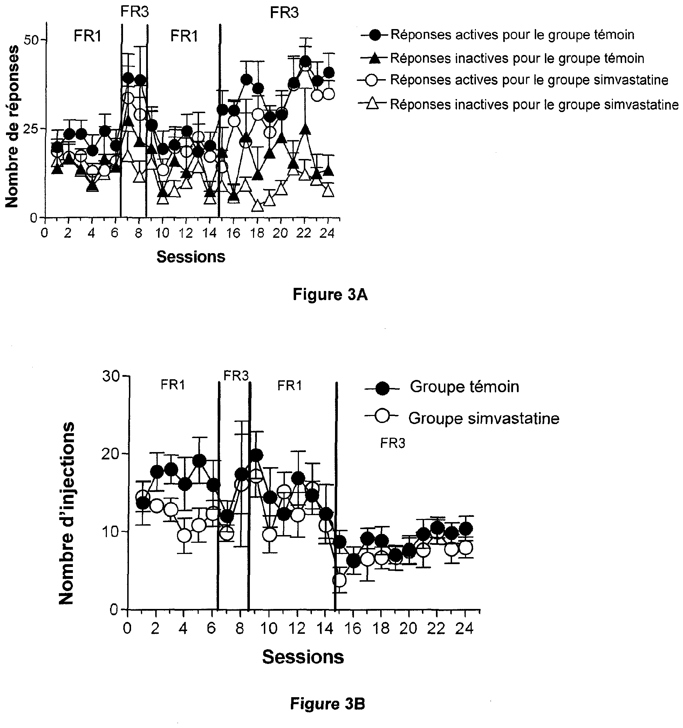

Les figures 3A et 3B représentent les niveaux moyens d'auto-administration de nicotine chez les rats qui recevront par la suite un traitement chronique d'une solution témoin (Groupe témoin) ou un traitement chronique de simvastatine (Groupe simvastatine). La figure 3A représente le nombre de réponses pour les orifices (*nose-pokes*) actifs et inactifs et la figure 3B représente le nombre d'injections de nicotine durant les 24 sessions d'auto-administration selon un programme de renforcement fixé à 1 ou 3 (FR1 ou FR3). Il est à noter que les traitements chroniques de solution témoin ou de simvastatine débutent le jour après la dernière session d'auto-administration et se terminent le jour précédent les tests de comportement de recherche de drogues.

### Figure 4

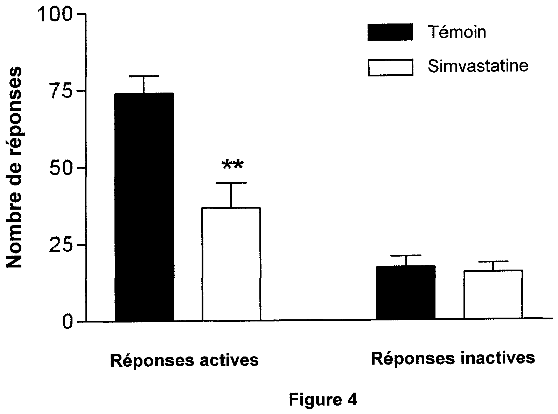

La figure 4 représente la diminution du comportement de recherche de nicotine chez les rats traités de manière chronique à la simvastatine. Le comportement de recherche de drogues est mesuré par le nombre de réponses dans les orifices (*nose-pokes*) actifs réalisées par les rats durant la session du test d'extinction de 1 h. Les réponses actives ne sont pas renforcées par une injection de nicotine. Le symbole ** indique une diminution significative des réponses du groupe simvastatine par rapport au groupe témoin.

### Figures 5A et 5B

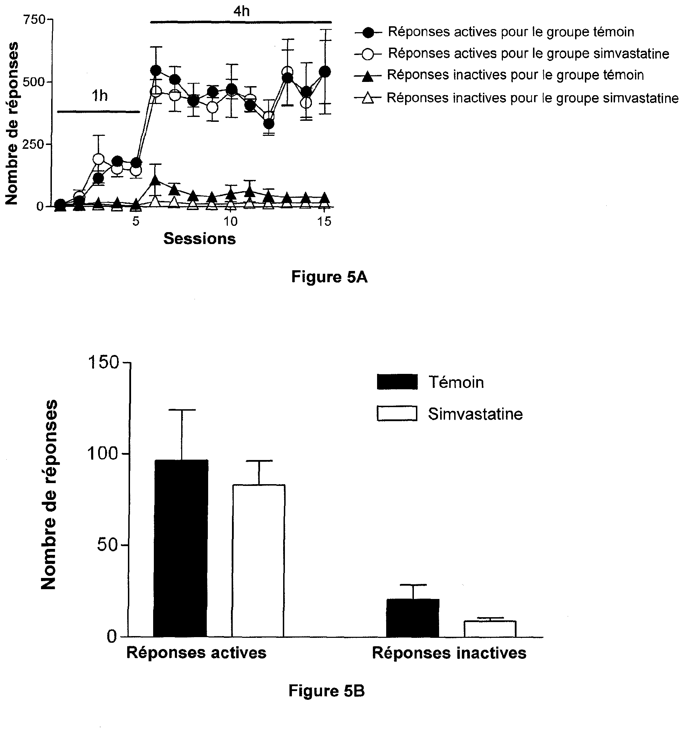

La figure 5A représente les niveaux moyens d'auto-administration de nourriture chez les rats qui recevront par la suite un traitement chronique d'une solution témoin (Groupe témoin) ou un traitement chronique de simvastatine (Groupe simvastatine) au cours de sessions de 1 heure ou de 4 heures. La figure 5B représente le nombre de réponses pour les orifices (*nose-pokes*) actifs et inactifs durant les 15 sessions d'auto-administration. Il est à noter que les traitements chroniques de solution témoin ou de simvastatine débutent le jour après la dernière session d'auto-administration et se terminent le jour précédent les tests de comportement de recherche de nourriture.

### Description détaillée de l'invention

Comme on l'entend ici, une « addiction » à une drogue se définit, notamment, par une consommation de drogue qui n'est plus contrôlée par l'individu et qui persiste malgré ses éventuelles conséquences négatives, présentes ou prévisibles, sur l'individu, en particulier chez un individu qui a conscience ou qui est informé des éventuelles conséquences négatives. Selon l'invention, les statines permettent d'éviter ou de limiter l'installation d'une addiction et d'atténuer ou d'éliminer une addiction déjà installée. Ainsi, en particulier, les statines permettent à l'individu dans une situation d'addiction à une drogue, de contrôler, et notamment d'arrêter, la consommation de la drogue.

Comme on l'entend ici, le « sevrage » de la consommation d'une drogue chez un individu se définit, en particulier, par l'arrêt de la consommation de la drogue chez un individu, notamment chez un individu en situation d'addiction vis-à-vis de la drogue. Selon l'invention, les statines facilitent le sevrage et permettent, notamment, de diminuer le risque ou la probabilité qu'un individu sevré ou abstinent, c'est-à-dire ayant arrêté de consommer une drogue, notamment une drogue vis-à-vis de laquelle il était en situation d'addiction, ne rechute, c'est-à-dire ne recommence à consommer la drogue.

En particulier, selon l'invention, les statines permettent de prévenir ou de traiter le désir de consommer une drogue, notamment chez un individu sevré d'une drogue vis-à-vis de laquelle il était en situation d'addiction ou dont il était dépendant. Le désir, souvent qualifié d'intense, de consommer une drogue est également désigné sous le nom anglo-saxon de *craving.*

De manière préférée, l'individu selon l'invention est un être humain. L'individu selon l'invention présente notamment une toxicomanie, une dépendance, ou se trouve dans un état d'addiction, vis-à-vis de la drogue selon l'invention.

Comme on l'entend ici, les drogues selon l'invention regroupent l'ensemble des composés susceptibles d'induire une dépendance ou une addiction chez un individu en consommant. Les drogues selon l'invention peuvent être notamment nommées « drogue d'addiction » ou « drogue d'abus ». La consommation de drogues selon l'invention peut être réalisée par tout type de voies, par exemple par ingestion, inhalation, ou encore injection sous-cutanée ou intraveineuse.

Les drogues selon l'invention peuvent notamment être des produits stupéfiants, des médicaments, du tabac ou de l'alcool.

De préférence, les drogues selon l'invention sont sélectionnées dans le groupe constitué de la cocaïne, du crack, du cannabis, de la morphine, des morphiniques, de l'héroïne, de l'ecstasy, du LSD, des amphétamines, de la kétamine, du tabac, et de l'alcool.

Comme cela apparaîtra clairement à l'homme du métier, l'addiction au tabac selon l'invention est généralement une addiction à la nicotine et l'addiction à l'alcool selon l'invention est généralement une addiction à l'éthanol.

Comme on l'entend ici, le terme « cannabis » regroupe l'ensemble ou n'importe lequel des cannabinoïdes psychoactifs, dont notamment le tétrahydrocannabinol (THC). Le terme « morphinique », quant à lui, désigne l'ensemble ou n'importe lequel des opioïdes psychoactifs ou des dérivés de la morphine psychoactifs.

De manière particulièrement préférée, la drogue selon l'invention est la nicotine, la cocaïne ou le crack, notamment la nicotine ou la cocaïne. Le terme de « cocaïne » regroupe, ou désigne indifféremment, la cocaïne en elle-même ainsi que ses différents sels, tels que le chlorhydrate de cocaïne.

Les statines, ou inhibiteurs de la HMG-CoA réductase, forment une classe médicamenteuse bien connue de l'homme du métier. Les statines sont notamment décrites par Smith et al. (2009) Drug class review: HMG-CoA reductase inhibitors (statins). Update 5 (http://www.ohsu.edu/drugeffectiveness/reports/final.cfm). Par ailleurs, une « statine » au sens de l'invention désigne aussi bien une statine en elle-même qu'un sel pharmaceutiquement acceptable d'une statine.

De préférence, la statine selon l'invention est sélectionnée dans le groupe constitué de la simvastatine, de l'atorvastatine, de la fluvastatine, de la lovastatine, de la pravastatine, et de la rosuvastatine.

De manière particulièrement préférée, la statine selon l'invention est la simvastatine.

Le composé additionnel destiné à la prévention ou au traitement de l'addiction à une drogue ou au sevrage de la consommation d'une drogue selon l'invention peut être de tout type, on préfère toutefois qu'il soit sélectionné dans le groupe constitué de la méthadone, de la buprénorphine, de la naltréxone, de l'acamprosate, du topiramate, du bupropion, du rimonabant, du disulfiram, du modafinil, du propranolol, du baclofène, et de l'ondansetron. Pour la composition pharmaceutique selon l'invention, le composé additionnel destiné à la prévention ou au traitement de l'addiction à une drogue ou au sevrage de la consommation d'une drogue selon l'invention est sélectionné dans le groupe constitué de la méthadone, de la buprénorphine, de la naltréxone, de l'acamprosate, du topiramate, du bupropion, du disulfiram, du modafinil, du propranolol, du baclofène, et de l'ondansetron. Par ailleurs, lorsque la drogue selon l'invention est le tabac, le composé additionnel destiné à la prévention ou au traitement de l'addiction à une drogue ou au sevrage de la consommation d'une drogue selon l'invention peut être la nicotine administrée autrement que par inhalation de fumées provenant de la combustion du tabac, notamment par voie transdermique, par exemple à l'aide d'un patch, par voie nasale, par exemple à l'aide d'un vaporisateur, ou par voie orale, par exemple à l'aide d'une gomme à mâcher. Ces composés additionnels sont notamment décrits dans O'Brien (2005) Am. J. Psychiatry 162:1423-1431.

En particulier, lorsque la drogue est la cocaïne ou le crack, le composé additionnel est de préférence le disulfiram, le topiramate, le modafinil, le propranolol, ou le baclofène. Lorsque la drogue est la nicotine, le composé additionnel est de préférence le bupropion ou le rimonabant, plus préférablement le bupropion.

Comme on l'entend ici l'expression « en combinaison » ou « produit de combinaison » signifie que la statine selon l'invention et le composé additionnel selon l'invention peuvent être associés au sein d'une même composition pharmaceutique, et donc être administrés ensemble, ou bien être administrés de manière séparée, c'est-à-dire selon des voies d'administration distinctes et/ou des régimes d'administration distincts, sous réserve que lorsqu'ils sont administrés de manière séparée les périodes d'activité respectives de la statine et du composé additionnel se recouvrent en totalité ou en partie.

Ainsi, lorsque la statine selon l'invention et le composé additionnel selon l'invention sont administrés de manière séparée, le composé additionnel sera de préférence administré dans les 24 heures, plus préférablement dans les 2 heures, et encore plus préférablement dans l'heure, suivant l'administration de la statine, et son administration sera éventuellement poursuivie les jours suivants. Réciproquement, la statine sera de préférence administrée dans les 24 heures, plus préférablement dans les 2 heures, et encore plus préférablement dans l'heure, suivant l'administration du composé additionnel, et son administration sera éventuellement poursuivie les jours suivants. Dans un autre mode de réalisation préférée de l'invention, lorsque la statine et le composé additionnel selon l'invention sont administrés de manière séparée, ils sont administrés essentiellement simultanément.

### Exemples

Les inventeurs ont utilisé des protocoles d'auto-administration chez le rat pour tester les effets curatifs de la simvastatine dans un modèle préclinique. Ces protocoles permettent de mimer au mieux la dépendance chez l'homme. En effet, dans les expériences d'auto-administration, les rats peuvent s'auto-injecter la drogue de façon intraveineuse en appuyant sur un levier ou en passant leur nez dans un orifice (*nose-poke*)*.* Ils contrôlent ainsi eux-mêmes leur consommation de drogue. Ce modèle est particulièrement représentatif de la situation chez l'homme car il inclut des facteurs de prise et de recherche de drogues identiques à ceux observés chez l'homme.

En particulier, parce que la rechute constituée par la prise de cocaïne ou de nicotine après une longue période d'abstinence est l'un des obstacles les plus difficiles pour guérir de l'addiction, les inventeurs se sont focalisés sur cette phase du cycle de l'addiction en utilisant un modèle de réinstallation d'un comportement de recherche de drogue après une période de sevrage forcé. Dans ce modèle, les animaux apprennent tout d'abord à s'auto-administrer la drogue et sont ensuite maintenus en phase d'abstinence durant une période donnée (de un jour à plusieurs mois), afin de mesurer la persistance du désir (*craving*) pour la drogue lors du test de rechute, durant lequel la drogue n'est pas disponible (Epstein et al. (2006) Psychopharmacology (Berl.) 189:1-16).

Ainsi, la logique de l'expérience est la suivante :
1) les rats acquièrent l'auto-administration dans des conditions similaires ;
2) pendant le sevrage de l'auto-administration, ils sont séparés et reçoivent une fois par jour pendant 20 jours une injection de simvastatine (1 mg/kg i.p) ou une solution témoin ;
3) ils sont testés dans un protocole de recherche de drogues dans lequel leurs réponses ne sont pas renforcées par la drogue.

Par ailleurs, les inventeurs ont étudié les effets de la simvastatine sur le comportement de recherche de nourriture afin d'évaluer la spécificité d'action de ce composé.

### Matériels et méthode

### Sujets

Des rats adultes mâles (11-12 semaines) Sprague-Dawley (Janvier, France), expérimentalement naïfs au début de l'étude sont placés dans l'animalerie avec contrôle de la température et du degré d'humidité, et sont maintenus dans un cycle de 12 heures lumière/obscurité (lumière active à 7 heures). Dès leur arrivée, les rats sont placés dans des cages par groupe de 3 durant une semaine, avant de débuter la chirurgie, consistant en l'implantation d'un cathéter dans la veine jugulaire. Après la chirurgie et durant le reste de l'expérimentation, les rats sont placés dans des cages de manière individuelle. Toutes les expérimentations se déroulent durant la phase "jour", en accord avec les régulations C.E. pour l'utilisation des animaux en recherche (86/609/EEC).

### Cathétérisation

Pour les procédures d'auto-administration de cocaïne, les cathéters (Solinas et al. (2003) J. Pharmacol. Exp. Ther. 306:93-102) sont implantés dans la veine jugulaire droite, dans des conditions stériles et sous anesthésie générale (injection intrapéritonéale (i.p) de kétamine (60 mg/kg) et de xylazine (10 mg/kg)). Un verrou en plastique de 20 mm est implanté dans le dos du rat en sous-cutané. Durant les sessions d'auto-administration, le cathéter est connecté à la pompe d'injection via un tube en métal qui s'attache sur le verrou en plastique situé dans le dos du rat. Les cathéters sont vérifiés avant et après chaque session avec 0,1 ml de solution saline.

### Appareil et procédures d'auto-administration de cocaïne.

Les équipements et les procédures d'auto-administration sont similaires à ceux décrits par Chauvet et al. (2009) Neuropsychopharmacology 34:2767-78.

Les expériences d'auto-administration se déroulent dans des cages Imetronic équipées d'orifices (*nose-pokes*) et contrôlées par des interfaces et logiciels Imetronic (Imetronic, Pessac, France ; www.imetronic.com).

Pour la cocaïne, 20 sessions d'auto-administration sont conduites en utilisant un programme de renforcement fixé à 1 (ou FR1), c'est-à-dire que durant les sessions, une seule réponse dans l'orifice actif délivre immédiatement une injection de cocaïne en intraveineuse (i.v.) (0,6 mg/injection). Durant l'injection, il y a activation de la lumière durant 5 secondes suivi d'une période d'extinction de 5 secondes pendant laquelle la chambre d'auto-administration est sombre et les réponses n'ont aucune conséquence. Il n'y a pas de conséquence à une réponse dans l'orifice inactif. Les réponses dans l'orifice actif et inactif sont enregistrées. Toutes les sessions d'auto-administration durent 3 heures.

Pour la nicotine, 24 sessions d'auto-administration sont conduites en utilisant un programme de renforcement fixé à 1 ou à 3 (FR1 et FR3), c'est-à-dire que durant les sessions, respectivement une ou trois réponses dans l'orifice actif délivrent une injection de nicotine en i.v. Les doses délivrées de nicotines sont 22.5 µg/injection ou 7.5 µg/injection. Durant l'injection, il y a activation de la lumière durant 5 secondes suivi d'une période d'extinction de 5 secondes pendant laquelle la chambre d'auto-administration est sombre et les réponses n'ont aucune conséquence. Il n'y a pas de conséquence à une réponse dans l'orifice inactif. Les réponses dans l'orifice actif et inactif sont enregistrées. Toutes les sessions d'auto-administration durent 1 ou 3 heures. Pendant les sessions 1-5, les rats peuvent s'auto-administrer une dose de nicotine de 22.5 µg/injection selon le protocole FR1 ; pendant les sessions 6-7, les rats peuvent s'auto-administrer une dose de nicotine de 22.5 µg/injection selon le protocole FR3 ; pendant les sessions 9-14, les rats peuvent s'auto-administrer une dose de nicotine de 7.5 µg/injection selon le protocole FR1 ; et pendant les sessions 15-24, les rats peuvent s'auto-administrer une dose de nicotine de 7.5 µg/injection selon le protocole FR3.

Pour la nourriture, 15 sessions d'auto-administration sont conduites en utilisant un programme de renforcement FR1 dans lequel une seule réponse dans l'orifice actif délivre immédiatement un granulé de nourriture de 45 mg. Durant la livraison de nourriture, il y a activation de la lumière durant 5 secondes suivie d'une période d'extinction de 5 secondes pendant laquelle la chambre d'auto-administration est sombre et les réponses n'ont aucune conséquence. Il n'y a pas de conséquence à une réponse dans l'orifice inactif. Les réponses dans l'orifice actif et inactif sont enregistrées. Les sessions d'auto-administration durent 1 ou 4 heures.

### Longue période d'abstinence et traitement chronique à la simvastatine

A la fin de la dernière session d'auto-administration, les rats sont divisés de manière pseudo-randomisée en 2 groupes ayant chacun des niveaux similaires d'auto-administration de drogue ou de nourriture : un groupe reçoit de la simvastatine à la dose de 1 mg/kg i.p. et l'autre groupe reçoit des volumes équivalents de solution témoin. Les rats sont maintenus abstinents durant 20 jours pendant lesquels ils reçoivent l'injection quotidienne de simvastatine ou de solution témoin (les injections sont réalisées entre 14 et 16 heures chaque jour).

Cette période d'abstinence permet le développement d'un phénomène appelé incubation du *craving*, ce qui se traduit par une augmentation du désir intense de consommer des drogues au cours du temps (Grimm et al. (2001) Nature 412:141-2).

De manière importante, la dernière injection de simvastatine est administrée le jour avant le test de comportement de recherche de drogue, afin d'éviter de potentiels effets directs de la drogue.

### Test de recherche de cocaïne

Après 20 jours de traitement à la simvastatine, les rats sont placés dans la pièce d'auto-administration et testés pour le comportement de recherche de drogue ou de nourriture dans une session d'extinction de 3 heures ou de 1 heure dans le cas de la nicotine. Les réponses dans les orifices (*nose-pokes*) actifs durant cette phase d'extinction produisent les mêmes *stimuli* (lumière et bruit de la pompe) qui étaient présents durant la phase d'auto-administration mais les seringues sont enlevées des pompes d'injection et la cocaïne ainsi que la nicotine ne sont pas délivrées. Pour la nourriture, les granulés sont retirés du distributeur et ne sont donc pas fournis. La session de 3 heures d'extinction est divisée en 3x1 h d'extinction (ou 1 h seulement pour la nicotine). Le nombre de réponses dans les orifices (*nose-pokes*) actifs est utilisé comme mesure du comportement de recherche de drogue.

### Drogues

La cocaïne est achetée chez COOPER (Cooperation Pharmaceutique Française, France; www.cooper.fr), la nicotine est achetée chez SIGMA/Aldrich, et la simvastatine est un don du laboratoire Ranbaxy, Inde. La cocaïne et la nicotine sont dissoutes dans une solution saline stérile (0,9%). La solution de simvastatine est préparée quotidiennement de la manière suivante : 5 mg de simvastatine sont dissouts dans 250 µl d'éthanol et 250 µl de tween 80 puis une solution saline stérile est ajoutée jusqu'à un volume final de 15 ml. Ainsi, une solution à 0,33 mg/ml de simvastatine est obtenue. Elle est injectée à un volume de 3 ml/kg pour une dose finale de 1 mg/kg. La solution témoin est identique mais n'inclue pas de simvastatine.

### Exemple 1 : le traitement chronique avec la simvastatine diminue la recherche de cocaïne

### Auto-administration de cocaïne avant le traitement à la simvastatine ou avec le témoin

A la fin de la période d'auto-administration chacun des 2 groupes devant respectivement recevoir la simvastatine et le témoin, présente des niveaux similaires d'auto-administration de cocaïne. Ainsi, le nombre de réponses actives et inactives ne diffère pas entre les futurs groupes simvastatine et témoin (Figures 1A et 1B).

### Le traitement chronique avec la simvastatine diminue la recherche de cocaïne

Les rats s'étant auto-administrés la cocaïne et ayant reçu par la suite la solution témoin pendant la période d'abstinence de 20 jours présentent un haut niveau de comportement de recherche de drogues durant le test (environ 350 réponses actives). En comparaison, des rats qui ne se sont jamais auto-administrés la cocaïne ne produisent pas plus de 20 réponses actives durant le même test.

Les rats s'étant auto-administrés la cocaïne et ayant reçu par la suite des injections quotidiennes de 1 mg/kg de simvastatine durant 20 jours d'abstinence montrent une réduction significative du comportement de recherche de drogue (environ 190 réponses actives soit une réduction de 45%) (Figures 2A et 2B).

### Exemple 2 : le traitement chronique avec la simvastatine diminue la recherche de nicotine

### Auto-administration de nicotine avant le traitement à la simvastatine ou avec le témoin

A la fin de la période d'auto-administration chacun des 2 groupes devant respectivement recevoir la simvastatine et le témoin, présente des niveaux similaires d'auto-administration de nicotine. Ainsi, le nombre de réponses actives et inactives ne diffère pas entre les futurs groupes simvastatine et témoin (Figures 3A et 3B).

### Le traitement chronique avec la simvastatine diminue la recherche de nicotine

Les rats s'étant auto-administrés la nicotine et ayant reçu par la suite la solution témoin pendant la période d'abstinence de 20 jours présentent un niveau élevé de comportement de recherche de drogues durant le test (environ 100 réponses actives). Les rats s'étant auto-administrés la nicotine et ayant reçu par la suite des injections quotidiennes de 1 mg/kg de simvastatine durant 20 jours d'abstinence montrent une réduction significative du comportement de recherche de drogue (environ 50%) (Figure 4).

### Exemple 3 : le traitement chronique avec la simvastatine n'affecte pas la recherche de nourriture

### Auto-administration de nourriture avant le traitement à la simvastatine ou avec le témoin

A la fin de la période d'auto-administration chacun des 2 groupes devant respectivement recevoir la simvastatine et le témoin, présente des niveaux similaires d'auto-administration de nourriture. Ainsi, le nombre de réponses actives et inactives ne diffère pas entre les futurs groupes simvastatine et témoin (Figure 5A).

### Le traitement chronique avec la simvastatine ne modifie pas la recherche de nourriture

Il n'y a pas de différence significative dans le comportement de recherche de nourriture des rats témoins et des rats ayant reçu de la simvastatine (Figure 5B).

En conséquence, le traitement chronique avec la simvastatine ne modifie pas le comportement de recherche de nourriture, ce qui suggère que la simvastatine agit spécifiquement sur les effets des drogues d'addiction et non sur ceux des récompenses naturelles.

## Revendications

1. Statine pour son utilisation dans la prévention ou le traitement de l'addiction à une drogue ou dans le sevrage de la consommation d'une drogue chez un individu.

2. Statine pour son utilisation selon la revendication 1, la statine étant sélectionnée dans le groupe constitué de la simvastatine, de l'atorvastatine, de la fluvastatine, de la lovastatine, de la pravastatine, et de la rosuvastatine.

3. Statine pour son utilisation selon la revendication 1 ou 2, la statine étant la simvastatine.

4. Statine pour son utilisation selon l'une des revendications 1 à 3, la drogue étant sélectionnée dans le groupe constitué de la cocaïne, du crack, du cannabis, de la morphine, des morphiniques, de l'héroïne, de l'ecstasy, du LSD, des amphétamines, de la kétamine, du tabac, notamment de la nicotine, et de l'alcool, notamment de l'éthanol.

5. Statine pour son utilisation selon l'une des revendications 1 à 4, la drogue étant la cocaïne ou la nicotine.

6. Statine pour son utilisation selon l'une des revendications 1 à 5, dans la prévention ou le traitement du désir de consommer une drogue.

7. Statine pour son utilisation selon l'une des revendications 1 à 6, en combinaison avec au moins un composé additionnel destiné à la prévention ou au traitement de l'addiction à une drogue ou au sevrage de la consommation d'une drogue.

8. Statine pour son utilisation selon la revendications 7, dans laquelle le composé additionnel est sélectionné dans le groupe constitué de la méthadone, de la buprénorphine, de la naltréxone, de l'acamprosate du topiramate, du bupropion, du rimonabant, du disulfiram, du modafinil, du propranolol, du baclofène, et de l'ondansetron.

9. Composition pharmaceutique comprenant à titre de substances actives :
- au moins une statine telle que définie dans l'une des revendications 1 à 3, et
- au moins un composé additionnel sélectionné dans le groupe constitué de la méthadone, de la buprénorphine, de la naltréxone, de l'acamprosate, du topiramate, du bupropion, du disulfiram, du modafinil, du propranolol, du baclofène, et de l'ondansetron, et
- éventuellement un véhicule pharmaceutiquement acceptable.

10. Composition pharmaceutique comprenant à titre de substances actives :
- au moins une statine telle que définie dans l'une des revendications 1 à 3, et
- au moins un composé additionnel sélectionné dans le groupe constitué de la méthadone, de la buprénorphine, de la naltréxone, de l'acamprosate, du topiramate, du bupropion, du rimonabant, du disulfiram, du modafinil, du propranolol, du baclofène, et de l'ondansetron, et
- éventuellement un véhicule pharmaceutiquement acceptable,
pour son utilisation dans la prévention ou le traitement de l'addiction à une drogue ou dans le sevrage de la consommation d'une drogue.

11. Produits comprenant :
- au moins une statine telle que définie dans l'une des revendications 1 à 3, et
- au moins un composé additionnel sélectionné dans le groupe constitué de la méthadone, de la buprénorphine, de la naltréxone, de l'acamprosate, du topiramate, du bupropion, du rimonabant, du disulfiram, du modafinil, du propranolol, du baclofène, et de l'ondansetron,
en tant que produit de combinaison pour une utilisation simultanée, séparée ou étalée dans le temps, dans la prévention ou le traitement de l'addiction à une drogue ou dans le sevrage de la consommation d'une drogue.

## Patentansprüche

1. Statin für seine Verwendung in der Prävention oder der Behandlung der Drogensucht oder der Entwöhnung des Drogenkonsums eines Individuums.

2. Statin für seine Verwendung nach Anspruch 1, wobei das Statin aus der Gruppe ausgewählt wird, die aus Simvastatin, Atorvastatin, Fluvastatin, Lovastatin, Pravastatin und Rosuvastatin besteht.

3. Statin für seine Verwendung nach Anspruch 1 oder 2, wobei das Statin Simvastatin ist.

4. Statin für seine Verwendung nach einem der Ansprüche 1 bis 3, wobei die Droge aus der Gruppe ausgewählt wird, die aus Kokain, Crack, Cannabis, Morphin, Opoide, Heroin, Ecstasy, LSD, Amphetamine, Ketamin, Tabak, insbesondere Nikotin und Alkohol, insbesondere Ethanol besteht.

5. Statin für seine Verwendung nach einem der Ansprüche 1 bis 4, wobei die Droge Kokain oder Nikotin ist.

6. Statin für seine Verwendung nach einem der Ansprüche 1 bis 5 in der Prävention oder der Behandlung des Verlangens, eine Droge zu konsumieren.

7. Statin für seine Verwendung nach einem der Ansprüche 1 bis 6 in Kombination mit mindestens einer Zusatzverbindung vorgesehen für die Prävention oder Behandlung einer Drogenabhängigkeit oder die Entwöhnung eines Drogenkonsums.

8. Statin für seine Verwendung nach Anspruch 7, bei dem die Zusatzverbindung aus der Gruppe ausgewählt wird, die besteht aus: Methadon, Buprenorphin, Naltrexon, Acamprostat, Topiramat, Bupropion, Rimonabant, Disulfiram, Modafinil, Propranolol, Baclofen und Ondansetron.

9. Pharmazeutische Verbindung, als aktive Substanzen umfassend:
- mindestens ein Statin, wie es in den Ansprüchen 1 bis 3 definiert ist, und
- mindestens eine Zusatzverbindung, die aus der Gruppe ausgewählt wird, die aus Methadon, Buprenorphin, Naltrexon, Acamprostat, Topiramat, Bupropion, Disulfiram, Modafinil, Propranolol, Baclofen und Ondansetron besteht, und
- gegebenenfalls einen pharmazeutisch verträglichen Träger.

10. Pharmazeutische Verbindung, als aktive Substanzen umfassend:
- mindestens ein Statin, wie es in den Ansprüchen 1 bis 3 definiert ist, und
- mindestens eine Zusatzverbindung, die aus der Gruppe ausgewählt wird, die aus Methadon, Buprenorphin, Naltrexon, Acamprostat, Topiramat, Bupropion, Rimonabant, Disulfiram, Modafinil, Propranolol, Baclofen und Ondansetron besteht, und
- gegebenenfalls einen pharmazeutisch verträglichen Träger, für ihre Verwendung in der Prävention oder der Behandlung der Drogensucht oder bei der Entwöhnung des Drogenkonsums.

11. Produkte, umfassend:
- mindestens ein Statin, wie es in einem der Ansprüche 1 bis 3 definiert ist, und
- mindestens eine Zusatzverbindung, die aus der Gruppe ausgewählt wird, die aus Methadon, Buprenorphin, Naltrexon, Acamprostat, Topiramat, Bupropion, Rimonabant, Disulfiram, Modafinil, Propranolol, Baclofen und Ondansetron besteht,
als Kombinationsprodukt für eine gleichzeitige, getrennte, oder über die Zeit verteilte Verwendung in der Prävention oder der Behandlung der Drogensucht oder bei der Entwöhnung des Drogenkonsums.

## Claims

1. Statin for use in the prevention or treatment of addiction to a drug or in the withdrawal from consumption of a drug in an individual.

2. Statin for use according to claim 1, the statin being selected from a group composed of simvastatin, atorvastatin, fluvastatin, lovastatin, pravastatin, and rosuvastatin.

3. Statin for use according to claim 1 or 2, the statin being simvastatin.

4. Statin for use according to one of claims 1 to 3, the drug being selected from the group composed of cocaine, crack, cannabis, morphine, opioids, heroin, ecstasy, LSD, amphetamines, ketamine, tobacco, particularly nicotine, and alcohol, particularly ethanol.

5. Statin for use according to one of claims 1 to 4, the drug being cocaine or nicotine.

6. Statin for use according to one of claims 1 to 5, in the prevention or treatment of the desire to consume a drug.

7. Statin for use according to one of claims 1 to 6, in combination with at least one additional compound intended for the prevention or treatment of addiction to a drug or withdrawal from the consumption of a drug.

8. Statin for use according to claim 7, wherein the additional compound is selected from a group composed of methadone, buprenorphine, naltrexone, acamprosate, topiramate, bupropion, rimonabant, disulfiram, modafinil, propranolol, baclofen, and ondansetron.

9. Pharmaceutical composition comprising, as active ingredients:
- at least one statin as defined in one of claims 1 to 3, and
- at least one additional compound selected from a group composed of methadone, buprenorphine, naltrexone, acamprosate, topiramate, bupropion, disulfiram, modafinil, propranolol, baclofen, and ondansetron, and
- possibly an acceptable pharmaceutical vehicle.

10. Pharmaceutical composition comprising, as active ingredients:
- at least one statin as defined in one of claims 1 to 3, and
- at least one additional compound selected from a group composed of methadone, buprenorphine, naltrexone, acamprosate, topiramate, bupropion, rimonabant, disulfiram, modafinil, propranolol, baclofen, and ondansetron, and
- possibly an acceptable pharmaceutical vehicle,
for use in the prevention or treatment of addiction to a drug or in the withdrawal from consumption of a drug.

11. Products comprising:
- at least one statin as defined in one of claims 1 to 3, and
- at least one additional compound selected from a group composed of methadone, buprenorphine, naltrexone, acamprosate, topiramate, bupropion, rimonabant, disulfiram, modafinil, propranolol, baclofen, and ondansetron,
as a combination product for use that is simultaneous, separate, or spread over time, in the prevention or treatment of addiction to a drug or in the withdrawal from consumption of a drug.
